# EUROPEAN PATENT SPECIFICATION

(11) **EP 1 691 802 B1**
(45) Date of publication and mention of the grant of the patent: **30.09.2009**
(21) Application number: 03751861.0
(22) Date of filing: 13.08.2003
(51) Int. Cl.: A61K 31/34

(54) **METHODS FOR THE AMELIORATION OF ENVIRONMENTAL OXIDANT STRESS AND THE REGULATION OF BENEFICIAL GENES**
VERFAHREN ZUR LINDERUNG VON UMWELTBEDINGTEM STRESS DURCH OXIDATIONSMITTEL UND REGULIERUNG VORTEILHAFTER GENE
PROCEDES POUR L'AMELIORATION DE STRESS OXYDANT ENVIRONNEMENTAL ET LA REGULATION DE GENES BENEFIQUES

(43) Date of publication of application: 23.08.2006
(73) Proprietor: The Ester C Company, Prescott AZ 86301 (US)
(72) Inventor: ROBINSON, Larry, E., Prescott, AZ 86301 (US); BERNAL, Samuel, D., Woodland Hills, CA 91367 (US)
(74) Representative: Bates, Philip Ian
(86) International application number: PCT/US2003/025638
(87) International publication number: WO 2005/018633

(56) References cited:
- US-A- 4 968 716
- US-B1- 6 468 980
- SHALU MENDIRATTA ET AL.: "Erythrocyte ascorbate recycling: antioxidant effects in blood" FREE RADICAL BIOLOGY & MEDICINE, vol. 24, no. 5, 1998, pages 789-797, XP002486258
- HIRAI N; KAWANO H; HIRASHIMA O; MOTOYAMA T; MORIYAMA Y; SAKAMOTO T; KUGIYAMA K; OGAWA H; NAKAO K; YASUE H: "Insulin resistance and endothelial dysfunction in smokers: effects of vitamin C." AMERICON JOURNAL OF PHYSIOLOGY. HEART AND CIRCULATORY PHYSIOLOGY, vol. 279, 2000, pages H1172-H1178, XP002486259
- DIETRICH MARION; BLOCK GLADYS; NORKUS EDWARD P; HUDES MARK; TRABER MARET G; CROSS CARROLL E; PACKER LESTER: "Smoking and exposure to environmental tobacco smoke decrease some plasma antioxidants and increase gamma-tocopherol in vivo after adjustment for dietary antioxidant intakes." AMERICAN JOURNAL OF CLINICAL NUTRITION, vol. 77, January 2003 (2003-01), pages 160-166, XP002486260
- VALKONEN M M; KUUSI T: "Vitamin C prevents the acute atherogenic effects of passive smoking." FREE RADICAL BIOLOGY & MEDICINE, vol. 28, no. 3, 2000, pages 428-436, XP002486261

## Description

This invention relates to new uses of compositions which include both a mineral ascorbate and at least one vitamin C metabolite.

More particularly, the invention concerns uses of such compositions for the amelioration of environmental oxidant stress and the regulation of beneficial genes.

In another respect, the invention pertains to new medical indications for administering such compositions.

According to still another aspect, the invention relates to uses of such compositions in the manufacture of compositions specially adapted to prevent and/or treat such indications.

These and other, further and more specific aspects of the invention will be apparent to those skilled in the art from the following detailed description, taken in conjunction with the working examples.

### BACKGROUND OF THE INVENTION

Vitamin C is an essential nutrient for humans, required for various functions including neurotransmitter synthesis, tyrosine metabolism, iron absorption, collagen synthesis and histamine catabolism. To optimize these cellular and biochemical functions, adequate levels of the vitamin need to be delivered to the cells. Importantly, vitamin C is essential for various white blood cell functions, including mobilization to sites of infection, engulfment and destruction of foreign particles, and phagocytosis of infectious organisms. Vitamin C is also important in white cell function in the process of wound healing.

The white cells appear to consume and release higher amounts of vitamin C in smokers, as well as in individuals exposed to stress, trauma, and operative procedures. The lower levels of vitamin C correlate with lower immune cell function. It has been suggested that the plasma level of vitamin C only reflects the vitamin in transit, on the way to be delivered to critical target cells. Thus, cellular accumulation and biochemical functions are important in determining the effects of administering different sources of vitamin C.

Accordingly, it would be desirable to identify particularly advantageous vitamin C compositions and methods of use, that can increase bioavailability of vitamin C and maintain cellular concentrations of vitamin C in humans, thereby to prevent medical indications stemming from inadequate cellular concentration of vitamin C and/or thereby to provide more effective treatment of medical indications by increasing cellular concentrations of vitamin C.

### Prior Art Vitamin C Compositions

Prior patents and patent applications, commonly owned with this application, describe compositions comprising a mineral ascorbate (e.g., calcium ascorbate) and vitamin C metabolites, such as the aldono-lactones and edible salts of aldonic acids (e.g., calcium threonate). See, e.g., US 4,822,816; US 4,968,716; and US 5,070,085, the disclosures of which are incorporated herein by reference. See , also, US 6,147,813. Other uses of such compositions, e.g., in cancer therapy, are also described in commonly-owned patent US 6,468,980.

### BRIEF DESCRIPTION OF THE INVENTION

It has now been discovered that such mineral ascorbate-vitamin C metabolite compositions, described in the above-identified commonly-owned patents and patent applications, have significant new preventive and/or therapeutic effects. These may include amelioration of the effects of environmental oxidants by intracellular and extracellular effects and the beneficial regulation of genes which have immunomodulatory activity.

Thus, according to one embodiment, the invention provides a composition for use in ameliorating environmental oxidant stress due to environmental oxidants, comprising: (1) a mineral ascorbate; and (2) at least one compound selected from the group consisting of: the aldono-lactones, aldono-lactides and edible salts of L-threonic acid, L-xylonic acid and L-lyxonic acid; a furanone, especially a vitamin-C derived furanone; threose; erythreose; 3-hydroxy kojic acid; and 5-hydroxy maltol.

According to another aspect, the invention provides a composition for use in ameliorating the effects of tobacco smoking, in which, preferably, the mineral ascorbate of such compositions is calcium ascorbate.

Another aspect of the invention provides a composition(s) for beneficial regulation of genes which combat anti-oxidant stress, and which, therefore, have implications for aging, macular degeneration, cell protection and immunity, including immunomodulatory genes, enzyme/redox genes, proteinase inhibitors and anti-inflammatory genes, preferably in which the mineral ascorbate of such compositions is calcium ascorbate and the vitamin C metabolite is calcium threonate.

### DETAILED DESCRIPTION OF THE INVENTION

The compositions used in the practice of the invention can be administered to humans by any of a variety of art-recognized techniques, including oral ingestion in a variety of dosage forms, intravenous or parenteral injection, transdermal absorption, inhalation and the like, in solid or liquid form. The total amounts administered per unit time and total amounts will vary with the particular condition to be treated or prevented, but will be an amount effective to or at least partially effective to achieve the objective desired. The selection of the particular dosage form, method of administration, dose rates and total doses administered will be selectable according to art-recognized techniques by those skilled in art, having regard for this disclosure.

### White Blood Cell Accumulation and Retention

An important step in arresting bacterial infection is the action of white cells to engulf and destroy foreign particles (phagocytosis). Studies in which human white blood cells have been incubated with ascorbic acid and bacteria indicate that the vitamin promotes phagocytic activity as well as chemotaxis, the movement of white cells to sites of infection. Thus, the stimulation of phagocytosis and lymphocyte motility appear to be dependent upon the vitamin C concentration outside the cells.

In other prior human studies, blood from healthy adults shows significant increases in neutrophil motility and lymphocyte transformation one hour after intravenous injection of vitamin C. Vitamin C supplementation has been associated with enhancement of human leukocyte function and intramuscular vitamin C injections (500 mg/ day for 1 month) were also found to improve lymphocyte proliferation in response to certain mitogens in vitro. A single 4 g dose of vitamin C given to healthy adults had a significant increase in serum interleukin-1 and total white blood cell count. Thus, vitamin C apparently acts to increase disease resistance by boosting the level and function of white blood cells.

Therefore, vitamin C accumulation in white blood cells is an ideal indication of vitamin C bioavailability, because of the great importance of white cells in disease prevention and health maintenance.

### Example 1 - White Blood Cell Study

Three vitamin C preparations and a placebo were tested to determine white blood cell bioavailability and pharmacokinetics in a double-blind, randomized, crossover study in human subjects. The three vitamin C and placebo compositions were
"Test A": ascorbic acid USP
"Test B": a mineral ascorbate (calcium ascorbate) + vitamin C metabolites (principally calcium threonate), in which the metabolites are produced *in situ* by a process such as disclosed in US Patent 4,822,816
"Test C": a mineral ascorbate (calcium ascorbate) + vitamin C metabolites (calcium threonate), manufactured by adding calcium threonate to calcium ascorbate.
"Test D": placebo
All three vitamin C compositions were standardized to 1000mg vitamin C equivalent per dose tablets.

A total of 15 subjects (all male between the ages of 21 and 50) were divided into three groups of five and were entered into a four-way crossover design study to minimize inter-subject variability, i.e., in which the subjects act as their own controls. Of the 15 subjects, nine were non-smokers and six were smokers. The groups were well matched in characteristics of age, weight and blood glucose levels. The average age was about 28 years for both groups, weight of about 79 kilos and glucose of about 130. The non-smoking group reported that they had no history of cigarette smoking at all. The smokers averaged ten cigarettes per day, ranging from six to fourteen cigarettes per day, for at least two weeks before and throughout the duration of the study.

Before the trials started, and between each of the four crossover phases, there was a 7-day wash out period where the subjects avoided foods containing vitamin C. The subjects were given a vitamin C dose or placebo in random sequence in a fasting state and blood was drawn at 0, 1, 2, 3, 4, 8, and 24 hours. Urine was also collected for the 24 hour period.

Vitamin C levels of the urine, plasma and white blood cells were determined by HPLC. The areas under the curve (AUC) for the ascorbic acid levels detected in white cells, plasma and urine was determined for each group using the trapezoidal rule (Selby, 1979). The data and the standard deviation was analyzed using repeated-measures analysis of variance (P<0.5). The statistical analysis was done using methods of Vinson and Bose, 1988..

Each of the subjects was assigned a code number identification assigned at random by the computer. The identities of the subjects were kept confidential, known only to the screening investigator. Only the code numbers were used to protect the anonymity of the subjects. The subjects were randomized to each of three different groups. The tablet sets were placed in sealed envelopes and coded so that the identity of the tablet given at any particular time was not known by the test subject or the investigators executing the tests.

Testing was performed according to the following protocol:
-7 days: All the subjects were instructed to avoid fruit juice, fruits, vegetables, cereals fortified with vitamins and vitamin supplements for 5 days before the test dose. The subjects, having been screened for any chronic and acute illnesses, were instructed to refrain from taking any medications, including analgesics, throughout the study.
-1 day: Complete 24-hour urine samples were collected on the date before dose administration.
-8 hours: The subjects were instructed to fast from 12 midnight, to appear at the test site in the early morning in a fasting state.
+0 day: An in-dwelling venous catheter (with heparin lock) was inserted for repeated sampling of blood to avoid repeated venipunctures. Baseline blood samples were obtained first, and then the test articles or placebo were administered. The subjects were then allowed to eat a uniform diet with low vitamin and caloric content.
0 hr Blood sample (pre-tablet administration)
+ 1 hr Blood sample (post-tablet administration)
+ 2 hr Blood sample (post-tablet administration)
+ 3 hr Blood sample (post-tablet administration)
+ 4 hr Blood sample (post-tablet administration)
+ 8 hr Blood sample (post-tablet administration)
+ 24 hr Blood sample (post-tablet administration)
   0 to + 24 hr - Complete urine collection on the day of the dose.
+ 1-6 days: The day after the first tablet, the test subjects resumed their previous low vitamin C diets. '
+ 7 days: The subjects returned to the test site to repeat the trial, receiving the second tablet. Again, blood samples were drawn at 1,2,3,4,6,8 and 24 hours after dose administration. Complete 24-hour urine samples were collected on the day of the dose.
+ 8-13 days: The day after the test dose (day 8), the test subjects resumed their previous low vitamin C diets.
+ 14 days: The subjects returned to the test site to repeat the trial, receiving the third tablet to continue the crossover study. Again, blood samples were drawn at 1,2,3,4,6,8 and 24 hours after dose administration.
+21 days: The subjects returned for the fourth tablet to the test site to repeat the trial, receiving a different tablet to continue the crossover study. Again, blood samples were drawn at 1,2,3,4,6,8 and 24 hours after dose administration.

Each subject took the tablet with 250 ml of water, and was able to drink as much water as they desired during the study period. The subjects continued to consume low-vitamin C foods throughout the duration of the study.

The tablet sets indicated the code number of the subject and the type of tablet encoded, so that it was not known by the test subject or the investigators executing the tests. In each of the three parts of the protocol, the subjects were asked to take the test tablet after overnight fasting. The subjects were randomized to one of three groups and the groups were rotated according to the order of tablets which consisted of: Test B-Test C-Test A-Test D.

Blood for white cells and plasma determinations were collected from the subjects at the specified times after ingestion of test tablets, into 5mL heparinized "Vacutainer" tubes. The tubes were labeled with the subject's number and the time of blood collection. The codes and the samples were collected in the laboratory, without any identification by the laboratory staff. The blood samples were kept in ice all throughout the collection and during white cell separation.

The samples of blood, collected from the subjects in tubes with heparin anticoagulant were kept in ice until processing. An aliquot was collected in citrated tubes for determinations of blood counts and cell volumes. After centrifugation at 1000 rpm for 15 minutes, the plasma in the upper layer were pipetted into a new tube kept in ice. The white layer or buff coat layer, was pipetted into fresh tubes and diluted 3x with a buffered saline solution.

The cell suspension was layered onto 2 volumes of Ficoll-Hypaque in conical centrifuge tubes. After centrifugation at 2000 rpm for 15 minutes, the white cell layer was pipetted into fresh centrifuge tubes, kept in ice. The white cell suspension was diluted with 5 ml of cold saline and centrifuged at 2000 rpm for 5 minutes. The pelleted cells were resuspended in 2 ml of cold saline for counting and extraction. The cell suspension was repelleted and suspended in 1 ml of 90% methanol containing 1.0 mM EDTA and stored at -70°C. The preserved samples were stable for several months, without degradation of the ascorbic acid. Total and differential counts of the white cells collected were obtained to determine the proportions of neutrophils, lymphocytes and monocytes in each of the samples.

For analysis of ascorbic acid content, the frozen suspensions of white blood cells were thawed and sonicated for 15 sec to disrupt the cells. The suspensions were centrifuged and the supernatant separated from the insoluble sediment. The samples were in ice up to the point of analysis. Aliquots of the cell suspension were analyzed on a Waters HPLC system and normalized to cell counts.

Measurement of ascorbic acid in white cells and plasma were deproteination using a column. All samples underwent a precolumn deproteination method using a hydroxyapatite cartridge (PCPure cartridge) eluted with 10 mM sodium phosphate buffer (pH 6.8) containing 2.5 mg/ml L-cysteine (Iwase and Ono, 1994). After preparation, the elutent solution was stored frozen. Prior to use it was thawed and kept on ice. The solution was stable for at 5°C for three days.

A Waters HPLC system with a UV detector was used to analyze the ascorbate levels in the different white cell, plasma and urine samples. Separations were carried out on C-18 columns (5-um bead; 4.6 mm diameter) used with a 3-cm C-18 guard column. The mobile phase consisted of 5% (v/v) acetonitrile, 1% (v/v) acetic acid, 1.8 mM tetrabutylammonium hydroxide, at pH 4.8 (adjusted with 1.0N NaOH. Absorbance at 265 nm by the UV detector was used to detect and quantitate the ascorbate in the effluent. At this wavelength, there was no significant absorbance by several metabolites of ascorbate, including dehydroascorbate. A range of 500-2000 pmoles of ascorbic acid solubilized in the EDTA/methanol was used as standards. The standardization was performed each time samples were run.

In the statistical analysis, "Area under the curve" (AUC) was calculated using the trapezoidal rule. The AUC was normalized by dividing by 24. This creates a time-weighted average in the original units.

A three-way repeated measures ANOVA was performed with Method (Plasma or WBC), Product (Test A-D compositions), and Smoker (Yes or No) as factors. The three-way interaction, Method*Product*Smoker, was in the model as well as the three two-way interactions Method*Product, Method*Smoker, and Product*Smoker. Carry-over terms were included along with a random effect for the order in which the products were received. Because there were significant differences in baseline vitamin C levels, the baseline value was included for each subject, effectively analyzing changes from baseline. Least Square Means (LSMeans) are presented with standard errors. These are means as estimated by the ANOVA model and will most likely be different from ordinary means due to the correlation structure in the model. A series of a priori contrasts were calculated and are presented with p-values testing for differences from zero.

The 3-way interaction was not significant (p=0.2568). The 2-way interaction between Product and Smoker was not significant (p=0.7384) indicating that differences between smokers and, non-smokers in vitamin C levels averaged over Plasma and WBC methods were not significantly different for all 4 products. There were significant differences between Method and Smoker (p<0.0001) and between Method and Product (p<0.0001).

There was no significant difference between the Test B and Test C (mineral ascorbate + metabolites) compositions. The release of vitamin C from the white blood cells of Test B-C subjects was significantly slower than the release of vitamin C from the white blood cells of Test A (ascorbic acid) subjects. This difference was significant in both smokers and non-smokers, although the difference was even greater in smokers. The white blood cells of Test B-C subjects contained 1.5 - 1.7 times and 1.3 - 1.5 times the level of vitamin C after 24 hours as ascorbic acid subjects (smokers and non-smokers respectively). This showed that Test B-C results in greater retention of vitamin C in white blood cells than ascorbic acid. There were no significant differences between Test B-C and ascorbic acid in plasma or white blood cell uptake of vitamin C.

Thus, the three different vitamin C preparations did not-lead to significant differences in plasma and white cell uptake of vitamin C. However, the two mineral ascorbate/metabolite compositions led to longer retention of vitamin C in the white cells, compared to plain ascorbic acid. The longer retention of vitamin C in white cells was apparent 24 hours after oral administration. The lowest levels of white cell vitamin C were observed among smokers, showing more rapid drops in vitamin C levels compared to non-smokers, after administration of ascorbic acid. Smokers retained significantly higher levels white blood cell vitamin C after administration of the mineral ascorbate/metabolite compositions.

Therefore, the cellular levels of vitamin C need to be assessed, in addition to plasma levels, when determining adequacy of vitamin C supplementation. The cellular levels and metabolic requirement for vitamin C can also be influenced by various factors, including stress and smoking status.

The most dramatic finding in this study was the markedly different kinetics of retention of vitamin C in white cells of smokers compared to non-smokers. The white cells of non-smokers exhibited a slow decline of vitamin C in their cells such that they still retained 70-80 percent of the cellular vitamin C 8 hours after administration of vitamin C. In contrast, white cells of smokers rapidly released vitamin C and only showed about 50-60 percent retention 8 hours after vitamin C dose. The initial kinetics of uptake, however, were not markedly different, showing peaks of WBC levels generally 2-4 hours after dose administration.

The retention kinetics of different preparations of vitamin C showed the greatest differences in white cells of smokers. In this study population, the two preparations of mineral ascorbate/metabolites showed between 1.5 to 1.7 times the level of vitamin C in white cells at 24 hours after vitamin doses. The differences ' between Test B-C and ascorbic acid were already well evident eight hours after the dose. The release of vitamin from white cells of smoking subjects was slower with the Test B-C compositions, compared with ascorbic acid. Among non-smokers, the Test B-C preparations showed between 1.3 to 1.5 times the level of vitamin in white cells 24 hours after the dose.

In contrast to the difference in white blood cell levels of vitamin C with the different preparations, there were no significant differences in plasma levels obtained at different times after oral administration of vitamin C. However, the plasma levels of smokers again differed markedly from those of non-smokers. Unlike the slow decline in plasma vitamin C levels of non-smokers, between 2 to 8 hours after dose administration, the plasma levels of smokers showed rapid decline of vitamin C between 4 to 8 hours after vitamin C administration.

Thus, smokers have different requirements for vitamin C supplementation than non-smokers and vitamin C derived from calcium ascorbate plus metabolites, is retained longer than ascorbic acid in white cells, particularly those isolated from smokers. These findings have further implications on the importance of maintaining vitamin C levels under conditions of exposure to primary smoke, second-hand smoke and other environmental oxidant stresses, because:
Functionally, white cells need adequate levels of vitamin C for optimal cellular function to respond to oxidant stress and yet, oxidant exposure itself appears to cause rapid depletion of intracellular vitamin C.
White blood cells of smokers also have increased adhesiveness to endothelial cells, which may contribute to acute and chronic circulatory abnormalities including atherogenesis. (Weber et al 1996 Circulation 93(8): 1488-1492) Dietary supplementation with up to 2g per day of vitamin C reverses the abnormal adhesion of white blood cells. (Motoyama et al 1997 Am J Physiol 273 (4.2): H1644-1650)
Cigarette smoking has direct effects on blood vessels by causing endothelial cell dysfunction and impairment of flow-dependent dilation of blood vessels which are reversed by vitamin C supplementation. (Motoyama, *supra*)

Accordingly, the compositions of the invention provide for amelioration of numerous deleterious effects caused by cigarette smoke and other environmental oxidant stresses.

In addition to the compositions which provide intracellular and extracellular effects on white blood cells, described above, which ameliorate the effects of environmental oxidants, it has now been discovered that compositions comprising a mineral ascorbate + vitamin C metabolites, also have important gene regulation effects which combat the effects of environmental oxidants.

### Example 2 - Gene Regulation

This study compares the effects of treating unstimulated human whole blood with a "Test A" composition (mineral ascorbate + vitamin C metabolites'), a "Test B" composition (calcium ascorbate USP) having equivalent vitamin C content, and a control composition (assay medium + 1% DMSO) on up-regulating or downregulating specific genes which have immunomodulatory effects. Each vitamin C test composition is evaluated in a concentration response (50, 100 and 250 µg/ml) and time course (2, 6 and 24 hour) format, using real time quantitative PCR.

Human whole blood was drawn into 10ml Vacutainer tubes with Sodium Heparin. Whole blood was utilized within 10-15 minutes of draw. After treatment with the Test compositions, whole blood nucleic acid extraction was performed using an ABI Prism® 6700 Automated Nucleic Acid Workstation. First strand cDNA was made from purified RNA samples. First Strand synthesis was performed on the 6700 Automated Nucleic Acid Workstation using PE's TAQMAN Reverse Transcription reagents. cDNA sample integrity was checked by QPCR analysis of 18S and β-action content using the ABI Prism® 7700 Sequence Detection System. cDNA from each sample was amplified for each target gene in a QPCR reaction using Applied Biosystems' 2X TaqMan® Universal PCR Master Mix and a unique primer/probe set for each specified target gene (including endogenous 18S controls). Target gene amplification was run in sets of four replicates on the ABI Prism 7700 Sequence Detection System.

### Results obtained are:

Test A and Test B exhibit differential gene expression responses as early as two hours across a broad concentration range (50-250 ug/ml).

A 250 ug/ml concentration response comparison of Test A and Test B in unstimulated whole blood at 2 hours shows clear directional (induction vs suppression) gene expression response differences of select genes, most notably, GRO1, HMOX, ICAM, IL15, IL18, IL1A, IL1B, IL1RN, JUN, PLAU, TIMP1 and TNF.

By 6 hours, there are clearly less directional gene expression response differences (such as CSF2, ICAM, IL18, TNF and VEGF) observed between Test A and Test B, however, significant magnitude differences are maintained particularly in GRO1, GRO2, HMOX, IL15, AL1A, IL1B, IL1RN, PI3, PLAU and PTGS2. Interestingly, Test A is more effective in the induction of 2 out of 3 enzyme/redox genes (see HMOX1, PLA2G7 and PTGS2)..

At 24 hours, Test A and Test B exhibit a very similar immunostimulatory response profile. Again, Test A appears to be more effective in the induction of some key genes such as HMOX (redox) and ILIRN (anti-inflammatory).

### Example 3 - Gene Regulation In Intestinal and Eye Tissues

Test compositions of calcium ascorbate + vitamin C metabolites ("Test A"), calcium ascorbate USP ("Test B"), containing 1 mM vitamin C equivalent in fetal bovine serum and a Control (fetal bovine serum only) are prepared. Human Caco 2 (intestinal cells) were selected because they contain transporters for vitamin C, and retinal pigment epithelium cells (RPE) are were selected for this study because oxidative stress contributes to aging and to several vision threatening diseases, including Age-related Macular Degeneration (AMD).

Comparative gene regulation studies were conducted with the test compositions and control using microarray techniques (8000 genes). Based on the microarray results, confirmatory PCR studies were conducted on 40 genes selected based on their regulation by Test A. These genes were either affected by Test A only, or were more strongly regulated by Test A than by Test B. The PCR results showed four genes to be functionally most promising with respect to pharmacological use of Test A to combat anti-oxidant stress and enhance immunity.

The two cell lines were grown to confluency in DMEM containing 10% fetal bovine serum. Prior to treating the cells, the serum-containing medium was removed and replaced with low serum medium containing either 1 mM vitamin C equivalent of Test A or Test B. Control cultured cells received no drug treatment. The cells were cultured for 48 hrs in 5% CO2 then harvested by centrifugation.

RNA was extracted from the control or drug treated cells by standard procedure and processes for hybridization using RNA labeling and amplification kits (Clontech) according to the manufacture's protocol. 50 mg of total RNA was used for probe synthesis after the quality and quantity of the RNA was assessed by capillary electrophoresis.

The probe was used to hybridize nylon arrays which contained specific oligonucleotides that represented 8000 different human genes. The membranes were hybridized overnight, washed to remove excess, unbound labeled probe, and then exposed for varying time periods to X-ray films. Labeled membranes were also exposed to a phosphoroimaging screen overnight and expression signals plotted using Clontech's Atlas Image 1.5 TM software and Atlas Navigator TM 1.0.

Global normalization was performed using every gene on the 8K array and a set of house keeping genes that are not usually significantly regulated between various treatments. A color-coded technique was used to distinguish differences in intensity of gene expression signals between two mRNA samples and data collected can be exported on spread sheet (Microsoft Excel). The array software package determines significant changes in gene expression, either up or down, after subtracting the background noise and using housekeeping genes to assign signal intensity ratios between the control and experimental samples.

Because of variations inherent in the microfabrication and oligonucleotide spotting on gene arrays, hybridization reactions, and gene homology characteristics, the array is considered semiquantitative. Therefore. the expression profile of a specific gene of interest obtained from the array should be reproduced by PCR or Western Blot analysis.

PCR was performed using standard procedure. The Genbank accession numbers were used to study the gene structure and homology with other genes in the data base. Primers were synthesized to non-homologous regions of the gene and used in PCR reaction. The reactions typically were run with an annealing temperature between 58-61° C for 30 cycles. The amplification products were run out on 4% agarose gel in TBE buffer and stained with ethidium bromide. A UV transilluminator was used to detect signals and verify gene regulation profile. A Polaroid camera was used for documentation of the data

The genes selected were based on their regulation profiles from the microarray data for Test A and their function in the human body with respect to oxidative stress, cell protection and immunity. For example:
DAD-1, which is necessary for cell survival in a mammalian cell line, is sufficient to suppress some programmed cell death. DAD-1 is an endogenous programmed cell death suppressor in Caenorhabditis elegans and vertebrates.
Heat shock proteins are proteins involved in the body's ability to cope with stress. HSP70 is a protein shown to be induced in the nervous system in response to ischemia (oxygen starvation, growth of abnormal blood vessels, death of neurons). It is a protein that may offer protection to neuronal cells against toxic stimuli. In addition HSP70 has been implicated in cell-mediated immune response.
Tumor translationally controlled protein 1 (TCPT1): Studies on breast cancer and leukemia show that inhibition of this gene by antisense cDNA or small ' RNA molecules suppressed the malignant phenotype of these tumors. The protein may be regulating cell cycle processes that control tumor metastasis. Thus, down regulation of TCPT1 by Test A could have significant pharmacological implications for some malignant tumors
IGF2 (Somatomedin A) has effect on metabolic processes. It is a growth promoting hormone especially with cartilage and connective tissue and is responsible for controlling such processes as dwarfism and obesity.

The results showed that:
Test A upregulated heat shock protein, (HSP70 , heat shock protein (Genbank # L12723) two-fold greater than Test B. HSP70 was not detected in control cell lines.
Test A alone upregulated DAD-1 (defender against cell death, Genbank # D15057). No expression for this gene was seen in Test B controls.
TCPT1 (tumor protein translationally controlled, Genbank #NM-003295) was downregulated by Test A only. This protein was expressed after treatment with Test B and was also found in abundance in the non-treated control.
Test A and Test B both upregulated IGF2 (insulin growth factor) at significant levels compared to the control (non-treated ) cultures which expressed undetectable levels of the gene.

Having described the invention in such terms as to enable those skilled in the art to understand and practice it and, having identified the presently preferred embodiments thereof, I claim:

## Claims

1. A composition for use in ameliorating oxidant stress due to environmental oxidants comprising:
a) A mineral ascorbate; and
b) At least one compound selected from the group consisting of the aldono-lactones, aldono-lactides and edible salts of an aldonic acid, a furanone, threose, erythreose, 3-hydroxy kojic acid and 5-hydroxy maltol.

2. A composition according to claim 1 for ameliorating the effects of tobacco smoking.

3. A composition according to claim 1 or cairn 2, wherein said mineral ascorbate is calcium ascorbate and said compound is calcium threonate.

4. A composition for use in beneficially regulating genes which combat oxidative stress comprising:
a) A mineral ascorbate; and
b) At least one compound selected from the group consisting of the aidono-lactones, aldono-lactides and edible salts of an aldonic acid, a furanone, threose, erythreose, 3-hydroxy kojic acid and 5-hydroxy maltol.

5. A composition according to claim 4 in which said mineral ascorbate is calcium ascorbate and in which said compound is calcium threonate.

6. A composition according to claim 4 or 5 for upregulating heat shock protein HSP70.

7. A composition according to claim 4 or 5 for upregulating the DAD-1 gene.

8. A composition according to claim 4 or 5 for downregulating the TCPT1 gene.

9. A composition according to claim 4 or 5 for regulating immunomodulatory genes.

10. Use of a composition comprising: (a) a mineral ascorbate; and (b) at least one compound selected from the group consisting of the aldono-lactones, aldono-lactides and edible salts of an aldonic acid, a furanone, threose, erythreose, 3-hydroxy kojic acid and 5-hydroxy maltol; in the manufacture of a medicament for ameliorating oxidant stress due to environmental oxidants.

11. Use of a composition comprising: (a) a mineral ascorbate; and (b) at least one compound selected from the group consisting of the aldono-lactones, aidono-lactides and edible salts of an aldonic acid, a furanone, threose, erythreose, 3-hydroxy kojic acid and 5-hydroxy maltol; in the manufacture of a medicament for beneficially regulating genes which combat oxidative stress.

## Patentansprüche

1. Zusammensetzung für die Verwendung bei der Linderung von Oxidansbelastung infolge von Umweltoxidantien, die Folgendes umfasst:
a) ein Mineralascorbat und
b) wenigstens eine Verbindung, die ausgewählt ist aus der Gruppe bestehend aus den Aldono-Lactonen, Aldono-Lactiden und essbaren Salzen einer Aldonsäure, einem Furanon, Threose, Erythreose, 3-Hydroxy-Kojisäure und 5-Hydroxymaltol.

2. Zusammensetzung nach Anspruch 1 zur Linderung der Auswirkungen des Tabakrauchens.

3. Zusammensetzung nach Anspruch 1 oder Anspruch 2, wobei das genannte Mineralascorbat Calciumascorbat ist und die genannte Verbindung Calciumthreonat ist.

4. Zusammensetzung für die Verwendung bei der vorteilhaften Regulierung von Genen, die oxidative Belastung bekämpfen, die Folgendes umfasst:
a) ein Mineralascorbat und
b) wenigstens eine Verbindung, die ausgewählt ist aus der Gruppe bestehend aus den Aldono-Lactonen, Aldono-Lactiden und essbaren Salzen einer Aldonsäure, einem Furanon, Threose, Erythreose, 3-Hydroxy-Kojisäure und 5-Hydroxymaltol.

5. Zusammensetzung nach Anspruch 4, wobei das genannte Mineralascorbat Calciumascorbat ist und die genannte Verbindung Calciumthreonat ist.

6. Zusammensetzung nach Anspruch 4 oder 5 zum Up-Regulieren des Hitzeschockproteins HSP70.

7. Zusammensetzung nach Anspruch 4 oder 5 zum Up-Regulieren des DAD-1 Gens.

8. Zusammensetzung nach Anspruch 4 oder 5 zum Down-Regulieren des TCPT1 Gens.

9. Zusammensetzung nach Anspruch 4 oder 5 zum Regulieren von Immunmodulationsgenen.

10. Verwendung einer Zusammensetzung, die Folgendes umfasst: (a) ein Mineralascorbat und (b) wenigstens eine Verbindung, die ausgewählt ist aus der Gruppe bestehend aus den Aldono-Lactonen, Aldono-Lactiden und essbaren Salzen einer Aldonsäure, einem Furanon, Threose, Erythreose, 3-Hydroxy-Kojisäure und 5-Hydroxymaltol, zur Herstellung eines Medikaments zur Linderung von Oxidansbelastung infolge von Umweltoxidantien.

11. Verwendung einer Zusammensetzung, die Folgendes umfasst: (a) ein Mineralascorbat und (b) wenigstens eine Verbindung, die ausgewählt ist aus der Gruppe bestehend aus den Aldono-Lactonen, Aldono-Lactiden und essbaren Salzen einer Aldonsäure, einem Furanon, Threose, Erythreose, 3-Hydroxy-Kojisäure und 5-Hydroxymaltol, zur Herstellung eines Medikaments zur vorteilhaften Regulierung von Genen, die oxidative Belastung bekämpfen.

## Revendications

1. Composition à utiliser pour améliorer le stress oxydant causé par des oxydants de l'environnement, comprenant :
a) Un ascorbate minéral; et
b) Au moins un composé sélectionné parmi le groupe consistant en aldonolactones, aldonolactides et des sels comestibles d'un acide aldonique, un furanone, thréose, érythréose, acide 3-hydroxy kojique et 5-hydroxy maltol.

2. Composition selon la revendication 1 pour améliorer les effets de fumer du tabac.

3. Composition selon la revendication 1 ou la revendication 2, dans laquelle ledit ascorbate minéral est de l'ascorbate de calcium et ledit composé est du thréonate de calcium.

4. Composition à utiliser pour réguler avantageusement des gènes qui combattent le stress oxydant, comprenant :
a) Un ascorbate minéral; et
b) Au moins un composé sélectionné parmi le groupe consistant en aldonolactones, aldonolactides et des sels comestibles d'un acide aldonique, un furanone, thréose, érythréose, acide 3-hydroxy kojique et 5-hydroxy maltol.

5. Composition selon la revendication 4, dans laquelle ledit ascorbate minéral est de l'ascorbate de calcium et dans laquelle ledit composé est du thréonate de calcium.

6. Composition selon la revendication 4 ou 5 pour réguler positivement la protéine de choc thermique HSP70.

7. Composition selon la revendication 4 ou 5 pour réguler positivement le gène DAD-1.

8. Composition selon la revendication 4 ou 5 pour réguler négativement le gène TCPT1.

9. Composition selon la revendication 4 ou 5 pour réguler des gènes immunomodulateurs.

10. Utilisation d'une composition comprenant : (a) un ascorbate minéral; et (b) au moins un composé sélectionné parmi le groupe consistant en aldonolactones, aldonolactides et des sels comestibles d'un acide aldonique, un furanone, thréose, érythréose, acide 3-hydroxy kojique et 5-hydroxy maltol; dans la fabrication d'un médicament pour améliorer le stress oxydant causé par des oxydants de l'environnement.

11. Utilisation d'une composition comprenant : (a) un ascorbate minéral; et (b) au moins un composé sélectionné parmi le groupe consistant en aldonolactones, aldonolactides et des sels comestibles d'un acide aldonique, un furanone, thréose, érythréose, acide 3-hydroxy kojique et 5-hydroxy maltol; dans la fabrication d'un médicament pour réguler avantageusement des gènes qui combattent le stress oxydant.
